Europäisches Patentamt

(19) European Patent Office

Office européen des brevets

(11) Numéro de publication: **0 302 765**
A1

(12) ## DEMANDE DE BREVET EUROPEEN

(21) Numéro de dépôt: 88401480.4

(22) Date de dépôt: 15.06.88

(51) Int. Cl.⁴: **C 12 Q 1/00**
C 12 Q 1/32, G 01 N 33/53,
G 01 N 33/573, A 61 K 35/52

(30) Priorité: 27.07.87 FR 8710619

(43) Date de publication de la demande:
08.02.89 Bulletin 89/06

(84) Etats contractants désignés:
AT BE CH DE ES FR GB GR IT LI LU NL SE

(71) Demandeur: **LABORATOIRES SOEKAMI
PHARMACEUTICAL RESEARCH
94 rue Edouard Vaillant
F-92300 Levallois-Perret (FR)**

(72) Inventeur: **Adda, Denis Henri
61 ter Route de Carrière
F-78400 Chatou (FR)**

**Fraisse, Clotilde
2 Avenue René Boylesve
F-75016 Paris (FR)**

**Macandain, Catherine
48 Boulevard Vital Bouhot
F-92200 Neuilly (FR)**

**Brossard, Pascal
14 Rue Cabanis
F-75014 Paris (FR)**

**Eyquem, André
24 Avenue Felix Faure
F-75015 Paris (FR)**

(74) Mandataire: **Hirsch, Marc-Roger
Cabinet Hirsch 34 rue de Bassano
F-75008 Paris (FR)**

(54) **Procédé de détermination de la présence de spermatozoïdes vivants et tests à cette fin.**

(57) La présente invention a pour objet un procédé nouveau de détermination de la présence de spermatozoïdes vivants dans le liquide spermatique. Elle se rapporte également aux tests permettant la mise en oeuvre dudit procédé.

Ce procédé de détermination de la présence de spermatozoïdes vivants consiste à préparer un support auquel adhère un agent de fixation de sites de membrane de spermatozoïdes humains vivants, à mettre ledit support au contact du liquide spermatique, puis à traiter le support par une solution aqueuse tamponnée contenant un substrat spécifique de l'activité lacticodéhydrogénase du spermatozoïde, et contenant un révélateur.

EP 0 302 765 A1

## Description

## PROCEDE DE DETERMINATION DE LA PRESENCE DE SPERMATOZOIDES VIVANTS ET TESTS A CETTE FIN

La présente invention a pour objet un procédé nouveau de détermination de la présence de spermatozoïdes vivants dans le liquide spermatique. Elle se rapporte également aux tests permettant la mise en oeuvre dudit procédé.

En fait, l'invention a plus particulièrement pour objet un procédé permettant la détermination quantitative des spermatozoïdes présents dans un échantillon de liquide spermatique.

On connait un certain nombre de procédés permettant la détermination de la présence de spermatozoïdes vivants dans un liquide spermatique. Ces procédés présentent l'inconvénient soit de ne pas permettre une détermination quantitative soit de ne la permettre que sous réserve de l'emploi d'appareillages compliqués, donc coûteux, nécessitant un personnel qualifié; en fait dans ce dernier cas, la mesure quantitative des spermatozoïdes est obtenue

. soit par différence entre deux opérations, à savoir une détermination du nombre de spermatozoïdes morts et vivants présents dans un échantillon et une détermination du nombre de spermatozoïdes morts dont la présence est révélée par coloration dans l'échantillon, ce qui implique deux déterminations,

. soit par comptage du nombre des spermatozoïdes vivants par une technique de révélation impliquant coloration directe et mesure de fluorescence, donc nécessitant un appareillage compliqué (microscope par fluorescence, etc).

De tels procédés et dispositifs sont entre autres décrits dans

. Belsey et Al, Laboratory Manual for the examination of human semen and semen-cervical mucus interaction".Press Concern Singapour (Publications OMS);

. Eliasson et Al, le chapitre "Analysis of semen" dans l'ouvrage "The Testis" EDit. Burger and Kretser 1981 p. 381-399;

. Jouannet et Al "Facteurs de fertilité humaine" Colloques Inserm. Ed. Spira et Jouannet, 1981, vol. 103 p. 73-90.

La présente invention a pour objet un procédé de détermination de la présence de spermatozoïdes vivants consistant à préparer un support neutre ou auquel adhère un agent de fixation de sites de membrane de spermatozoïdes humains vivants, à mettre ledit support au contact du liquide spermatique, puis à traiter le support par une solution aqueuse tamponnée contenant un substrat spécifique de l'activité lacticodéhydrogénase du spermatozoïde, et contenant un révélateur.

Selon une forme d'exécution du procédé de l'invention, le support est un élément rigide, tel plaque, coupelle, tube, batonnet, bille, microsphère, ou un élément semi-rigide ou souple, tel membrane, papier, bandelette, etc.

Selon une autre forme d'exécution du procédé de l'invention, le support est formé de polystyrène, polypropylène, polyéthylène, chlorure de polyvinyle, cellulose, nylon, etc.

Selon une première forme d'exécution du procédé de l'invention, l'agent de fixation de sites de membrane de spermatozoïdes vivants est un agent immunologique consistant en un anticorps spécifique des spermatozoïdes, tels les anticorps monoclonaux ou polyclonaux ou des associations d'anticorps monoclonaux.

Selon une seconde forme d'exécution du procédé de l'invention, l'agent de fixation de sites de membrane de spermatozoïdes vivants est une lectine fixant des sucres de surface des spermatozoïdes.

Selon une autre forme d'exécution du procédé de l'invention, le support est une surface neutre, non activée, poreuse, souple, sans agent de fixation des sites de membranes des spermatozoides.

Selon une autre forme d'exécution encore du procédé de l'invention, l'agent de fixation précité est fixé sur le support par adsorption due au contact dudit support avec une solution dudit agent suivie d'un traitement de saturation de la surface puis d'un séchage à température et pendant une durée définis.

Selon une forme de réalisation du procédé de l'invention, le révélateur comprend une nicotinamide adénine dinucléotide (NAD), un réducteur et un colorant.

Selon une autre forme de réalisation du procédé de l'invention, le réducteur consiste en phénazineméthosulfate (PMS). Ce réducteur est destiné à provoquer le déplacement d'atomes d'hydrogène vers le colorant.

Selon une autre forme encore de réalisation du procédé de l'invention, le colorant est le nitrobleu de tétrazolium (NBT).

Selon une forme d'exécution du procédé de l'invention, le substrat de l'activité lacticodéhydrogénase est un L-lactate de métal alcalin, de préférence, de sodium ou de lithium.

Selon une autre forme d'exécution du procédé de l'invention, le substrat de l'activité lacticodéhydrogénase est un substrat spécifique de l'isoenzyme LDH-C4 ou LDH-X, tel l'acide cétohexanoïque.

Selon une autre forme encore d'exécution du procédé de l'invention, le substrat est celui d'un enzyme essentiel du spermatozoïde, tel glucose-6-phosphate déhydrogénase ou 6-phosphogluconate déhydrogénase.

La présente invention a également pour objet un test pour la mise en oeuvre du procédé précité consistant en un kit comprenant d'une part un support neutre ou auquel adhère un agent de fixation de sites de membrane de spermatozoïdes humains vivants, et d'autre part une solution aqueuse tamponnée contenant un substrat spécifique de l'activité lacticodéhydrogénase du spermatozoïde, et contenant un révélateur. De préférence, le support est un élément rigide tel plaque, coupelle, tube, batonnet, bille, microsphère, ou un élément semi-rigide ou souple, tel membrane, papier, bandelette, etc. et est formé de polystyrène, polypropylène, polyéthylène, chlorure de polyvinyle, cellulose, nylon, etc.

Selon un mode de réalisation du test de l'invention, l'agent de fixation de sites de membrane de spermatozoïdes vivants est un agent immunologique consistant en un anticorps spécifique des spermatozoïdes, tels les anticorps monoclonaux ou polyclonaux ou des associations d'anticorps monoclonaux.

Selon un autre mode de réalisation du test de l'invention, l'agent de fixation de sites de membrane de spermatozoïdes vivants est une lectine fixant des sucres de surface des spermatozoïdes.

Selon une forme d'exécution du test de l'invention, le support est une surface neutre, non activée, poreuse, souple, sans agent de fixation des sites de membranes des spermatozoïdes.

Selon une autre forme d'exécution du test de l'invention, le révélateur comprend une nicotinamide adénine dinucléotide (NAD), un réducteur et un colorant. De préférence, le réducteur consiste en phénazine-méthosulfate (PMS), le colorant est le nitrobleu de tétrazolium (NBT) et le substrat de l'activité lacticodéhydrogénase est un L-lactate de métal alcalin, de préférence, de sodium ou de lithium, un substrat spécifique de l'isoenzyme LDH-C4 ou LDH-X, tel l'acide cétohexanoïque ou un substrat de l'activité glucose-6-phosphate déhydrogénase ou de l'activité 6-phosphogluconate déhydrogénase.

D'autres buts et avantages de l'invention apparaîtront à la lecture de la description suivante donnée à titre non limitatif.

### Exemple 1

Dans un tube en polystyrène ayant 7,5 cm de long et 1,2 cm de diamètre, on verse une solution d'un anticorps monoclonal spécifique des spermatozoïdes. Après 60 minutes de contact, on retire ladite solution du tube, on verse dans le tube une solution bovine à 2% puis après lavage à l'aide d'un tampon TRIS, sèche le tube à une température de 45°C pendant 15 minutes.

On prépare, par ailleurs, une solution aqueuse tamponnée contenant 1 mmole de nicotinamide adénine dinucléotide, 0,1 mmole de nitrobleu de tétrazolium, 0,054 mmole de phénazine méthosulfate, 5 mmoles d'acide cétohexanoïque, 50 mmoles de TRIS, 150 mmoles de NaCl et de l'eau (qsp 1 litre).

### Exemple 2 et 3

On prépare les mêmes tube et solution aqueuse tamponnée que dans l'exemple 1, sinon que la solution d'anticorps spécifique est une solution d'anticorps polyclonaux spécifiques (exemple 2) et d'un mélange d'anticorps monoclonaux (exemple 3).

### Exemples 4 à 6

On prépare les mêmes tube et solution aqueuse tamponnée que dans les exemples 1 à 3 respectivement, sinon que la solution aqueuse tamponnée préparée comporte 6 mmoles de L-lactate de sodium.

### Exemples 7 à 9

On prépare les mêmes tube et solutions aqueuses tamponnées que dans les exemples 4 à 6 respectivement, sinon que le L-lactate de sodium est remplacé par 5,9 mmoles de L-lactate de lithium.

### Exemples 10 à 12

On prépare les mêmes tube et solution aqueuse tamponnée que dans l'exemple 1, sinon que le tube est un tube en polypropylène, en polyéthylène et en chlorure de polyvinyle respectivement.

### Exemples 13 à 21

Au lieu des tubes employés dans les exemples 1 à 9, on emploie une plaque standard de microtitration à base de polystyrène comportant 96 puits à fond plat ayant les dimensions suivantes 12,5 cm x 8,5 cm et présentant une épaisseur de 0,15 cm.

### Exemples 22 à 30

Au lieu des tubes employés dans les exemples 1 à 9, on emploie une membrane à base de nylon microporeux neutre ayant une surface de 15 mm de diamètre présentant une épaisseur de 150 microns, permettant une filtration verticale.

### Essais A1 à L1

On effectue une série de tests A1 à L1 sur les ensembles tube-solution aqueuse tamponnées des exemples 1 à 12 respectivement.

Pour mettre en oeuvre ces tests, on verse dans le tube, 0,5 cm³ de liquide spermatique. Après 15 minutes de contact entre le liquide spermatique et la paroi des tubes, on jette le liquide spermatique puis rince le tube. On verse ensuite dans les tubes les solutions aqueuses tamponnées correspondantes.

On obtient une coloration bleue violacée.

On effectue une série de tests témoins AB1 à LB1, le liquide spermatique étant remplacé par un volume correspondant de liquide spermatique dont les spermatozoïdes ont été tués par des cycles de congélation/décongélation classiques.

On n'obtient aucune coloration.

### Essais A2 à I2

On effectue des tests A2 à I2 identiques aux tests A1 à I1 sur les ensembles plaque-solution aqueuse tamponnée des exemples 13 à 21 respectivement.

De même, on effectue une série de tests AB2 à JB2 sur lesdites plaques.

### Essais A3 à C3

On dépose 0,5 cm³ de liquide spermatique sur la surface à base de nylon souple poreux; une solution de lavage permettant d'éliminer le plasma séminal, les spermatozoïdes restant fixés dans les mailles du nylon poreux. Les révélateurs sont les mêmes que ceux indiqués dans les exemples 1 ou 7 à 9.

<u>Lecture des tests</u>

La lecture des tests peut-être faite optiquement à l'oeil, au réflectomètre au spectrophotomètre dans le rayonnement visible ou ultraviolet.

On constate, pour les essais A1 à L1, une forte coloration du contenu des tubes, facile à déterminer par lecture visuelle et susceptible d'être quantifiée à l'aide d'un réflectomètre. Pour les essais A2 à I2, il est plus difficile de faire une lecture visuelle du test et il est préférable d'opérer par lecture optique à l'aide d'un spectrophotomètre de tupe MULTISKAN (dénomination commerciale de Titertek) par exemple ou un réflectomètre. Pour les essais A3 à C3, est obtenue une forte coloration susceptible d'être déterminée par lecture visuelle; l'emploi d'un réflectomètre permet de quantifier cette coloration.

Pour permettre une analyse quantitative, on utilise une échelle de coloration des bleus violacés obtenus. Par étalon nage on obtient un nuancier des colorations obtenues auquel on se réfère pour déterminer la concentration en spermatozoïdes vivants présents et on rapporte celle-ci au volume total de l'éjaculat. La détermination du nombre de spermatozoïdes vivants n'est pas suffisante pour déterminer la fertilité du liquide spermatique, la détermination de conditions supplémentaires telles que motilité du spermatozoïde étant entre autres nécessaire.

**Revendications**

1.- Procédé de détermination de la présence de spermatozoïdes vivants consistant à préparer un support neutre ou auquel adhère un agent de fixation de sites de membrane de spermatozoïdes humains vivants, à mettre ledit support au contact du liquide spermatique, puis à traiter le support par une solution aqueuse tamponnée contenant un substrat spécifique de l'activité lacticodéhydrogénase du spermatozoïde ou d'une autre enzyme essentielle du spermatozoïde et contenant un révélateur.

2.- Procédé selon la revendication 1, caractérisé en ce que le support est un élément rigide tel plaque, coupelle, tube, batonnet, bille, microsphère, ou un élément semi-rigide ou souple, tel membrane, papier, bandelette.

3.- Procédé selon la revendication 1 ou 2, caractérisé en ce que le support est formé d'une matière à base entre autre de polystyrène, polypropylène, polyéthylène, chlorure de polyvinyle, cellulose, nylon.

4.- Procédé selon une quelconque des revendications 1 à 3, caractérisé en ce que l'agent de fixation de sites de membrane de spermatozoïdes vivants est un agent immunologique consistant en un anticorps spécifique des spermatozoïdes, tels les anticorps monoclonaux ou polyclonaux ou des association d'anticorps monoclonaux.

5.- Procédé selon une quelconque des revendications 1 à 3, caractérisé en ce que l'agent de fixation de sites de membrane de spermatozoïdes vivants est une lectine fixant des sucres de surface des spermatozoïdes.

6.- Procédé selon une quelconque des revendications 1 à 3, caractérisé en ce que le support est une surface neutre, non activée, poreuse, souple, sans agent de fixation des sites de membranes des spermatozoïdes.

7.- Procédé selon une quelconque des revendications 1 à 6, caractérisé en ce que l'agent de fixation précité est fixé sur le support par adsorption due au contact dudit support avec une solution dudit agent suivie d'un traitement de saturation de la surface puis d'un séchage à température et pendant une durée définis.

8.- Procédé selon une quelconque des revendications 1 à 7, caractérisé en ce que le révélateur comprend une nicotinamide adénine dinucléotide (NAD), un réducteur et un colorant.

9.- Procédé selon une quelconque des revendications 1 à 8, caractérisé en ce que le réducteur consiste en phénazineméthosulfate (PMS).

10.- Procédé selon une quelconque des revendications 1 à 9, caractérisé en ce que le colorant est le nitrobleu de tétrazolium (NBT).

11.- Procédé selon une quelconque des revendications 1 à 9, caractérisé en ce que le substrat de l'activité lacticodéhydrogénase est un L-lactate de métal alcalin, de préférence, de sodium ou de lithium.

12.- Procédé selon une quelconque des revendications 1 à 10, caractérisé en ce que le substrat de l'activité lacticodéhydrogénase est un substrat spécifique de l'isoenzyme LDH-C4 ou LDH-X, tel l'acide cétohexanoïque.

13.- Procédé selon une quelconque des revendications 1 à 10, caractérisé en ce que le substrat est celui d'une enzyme essentielle du spermatozoïde, tel que glucose-6-phosphate déhydrogénase ou 6-phosphogluconate déhydrogénase.

14.- Test pour la mise en oeuvre du procédé selon une quelconque des revendications 1 à 13, caractérisé en ce qu'il consiste en un kit comprenant d'une part un support auquel adhère un agent de fixation de sites de membrane de spermatozoïdes humains vivants, et, d'autre part, une solution aqueuse tamponnée contenant un substrat spécifique de l'activité lacticodéhydrogénase du spermatozoïde ou d'une autre enzyme essentielle du spermatozoïde, et contenant un révélateur.

15.- Test selon la revendication 14, caractérisé en ce que le support est un élément rigide tel plaque, coupelle, tube, batonnet, bille, microsphère, ou un élément semi-rigide ou souple, tel membrane, papier, bandelette.

16.- Test selon la revendication 14 ou 15, caractérisé en ce que le support est formé d'une matière consistant entre autre de polystyrène, polypropylène, polyéthylène, chlorure de polyvinyle, cellulose, nylon.

17.- Test selon une quelconque des revendications 14 à 16, caractérisé en ce que l'agent de fixation de sites de membrane de spermatozoïdes vivants est un agent immunologique consistant en un anticorps spécifique des spermatozoïdes, tels les anticorps monoclonaux ou polyclonaux ou des association d'anticorps monoclonaux.

18.- Test selon une quelconque des revendications 14 à 17, caractérisé en ce que l'agent de fixation de sites de membrane de spermatozoïdes vivants est une lectine fixant des sucres de surface des spermatozoïdes.

19.- Test selon une quelconque des revendications 14 à 16, caractérisé en ce que le support est une surface neutre, non activée, poreuse, souple, sans agent de fixation des sites de membranes des spermatozoïdes.

20.- Test selon une quelconque des revendications 14 à 19, caractérisé en ce que l'agent de fixation précité est fixé sur le support par adsorption due au contact dudit support avec une solution dudit agent suivie d'un traitement de saturation de la surface puis d'un séchage à température et pendant une durée définis.

21.- Test selon une quelconque des revendications 14 à 20, caractérisé en ce que le révélateur comprend une nicotinamide adénine dinucléotide (NAD), un réducteur et un colorant.

22.- Test selon une quelconque des revendications 14 à 21, caractérisé en ce que le réducteur consiste en phénazineméthosulfate (PMS).

23.- Test selon une quelconque des revendications 14 à 22, caractérisé en ce que le colorant est le nitrobleu de tétrazolium (NBT).

24.- Test selon une quelconque des revendications 14 à 23, caractérisé en ce que le substrat de l'activité lacticodéhydrogénase est un L-lactate de métal alcalin, de préférence, de sodium ou de lithium.

25.- Test selon une quelconque des revendications 14 à 23, caractérisé en ce que le substrat de l'activité lacticodéhydrogénase est un substrat spécifique de l'isoenzyme LDH-C4 ou LDH-X, tel l'acide cétohexanoïque.

26.- Procédé selon une quelconque des revendications 14 à 23, caractérisé en ce que le substrat est celui d'une enzyme essentielle du spermatozoïde, tel que glucose-6-phosphate déhydrogénase ou 6-phosphogluconate déhydrogénase.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | FR-A-2 341 865 (LABORATOIRE DE RECHERCHE API) * En entier * | 1-3,6,7,12,14-16,19,25 | C 12 Q 1/00 C 12 Q 1/32 G 01 N 33/53 G 01 N 33/573 A 61 K 35/52 |
| A | BIOLOGICAL ABSTRACTS, vol. 71, no. 10, 1981, page 7340, résumé no. 70005, Philadelphia, US; M. SHIGETA et al.: "Sperm-immobilizing monoclonal antibody to human seminal plasma antigens", & CLIN. EXP. IMMUNOL. 42(3), 458-462, 1980 * Résumé * | 1,4,16 | |
| Y | CLINICAL CHEMISTRY, vol. 29, no. 8, août 1983, pages 1518-1521, Washington, US; G.P. BUTRIMOVITZ et al.: "Micromethod for determination of lactate dehydrogenase isoenzyme C4 activity in human seminal plasma" * En entier * | 1-3,6,7,12,14-16,19,25 | |
| A | CHEMICAL ABSTRACTS, vol. 94, no. 15, 13 avril 1981, page 255, résumé no. 116543z, Columbus, Ohio, US; N. ZIMMERMANN et al.: "A polyacrylamide gel method for the cytochemical demonstration of glucose-6-phosphate dehydrogenase activity in mouse sperm", & ACTA HISTOCHEM. 1981, 68(2), 227-30 * Résumé * ---             -/- | 13,26 | DOMAINES TECHNIQUES RECHERCHES (Int. Cl.4) C 12 Q G 01 N A 61 K |

Le présent rapport a été établi pour toutes les revendications

| Lieu de la recherche | Date d'achèvement de la recherche | Examinateur |
|---|---|---|
| LA HAYE | 01-08-1988 | HITCHEN C.E. |

EPO FORM 1503 03.82 (P0402)

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | CHEMICAL ABSTRACTS, vol. 67, no. 21, 20 novembre 1967, page 9227, résumé no. 98261s, Columbus, Ohio, US; J.P. FARRIAUX et al.: "Identification of human sperm by demonstrating lactic dehydrogenase fraction X", & LILLE MED. 12(6), 687-93(1967) * Résumé * | 1,12,25 | |
| A | CHEMICAL ABSTRACTS, vol. 98, no. 21, 23 mai 1983, page 356, résumé no. 175817x, Columbus, Ohio, US; R.N. PETERSON et al.: "Characterization of boar sperm plasma membranes by two-dimensional PAGE and isolation of specific groups of polypeptides by anion-exchange chromatography and lectin affinity chromatography", & J. ANDROL. 1983, 4(1), 71-81 * Résumé * | 5,18 | |
| A | GB-A-1 318 568 (A.P. FOSKER et al.) * En entier * | 1,8-11, 21-24 | DOMAINES TECHNIQUES RECHERCHES (Int. Cl.4) |

Le présent rapport a été établi pour toutes les revendications

| Lieu de la recherche | Date d'achèvement de la recherche | Examinateur |
|---|---|---|
| LA HAYE | 01-08-1988 | HITCHEN C.E. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
..............
& : membre de la même famille, document correspondant

EPO FORM 1503 03.82 (P0402)